# EUROPÄISCHE PATENTSCHRIFT

(11) **EP 0 920 837 B1**
(45) Veröffentlichungstag und Bekanntmachung des Hinweises auf die Patenterteilung: **31.03.2004**
(21) Anmeldenummer: 99103138.6
(22) Anmeldetag: 17.01.1997
(51) Int. Cl.: A61B 17/68

(54) **Mittel zur Fixierung eines kalottenförmigen Schädelkapselsegments**
Device for securing a cranial piece
Elément de fixation d'un fragment de la calotte cranienne

(30) Priorität: 03.02.1996 DE 19603887
(43) Veröffentlichungstag der Anmeldung: 09.06.1999
(62) Teilanmeldung aus: 97100715.8
(73) Patentinhaber: Lerch, Karl-Dieter, Dr. med., D-58452 Witten (DE)
(72) Erfinder: Lerch, Karl-Dieter, Dr. med., D-58452 Witten (DE)
(74) Vertreter: Hoeger, Stellrecht & Partner

(56) Entgegenhaltungen:
- US-A- 4 802 477

## Beschreibung

Die Erfindung betrifft eine Vorrichtung zum Fixieren eines aus der Schädelkapsel zum Zwecke eines operativen Eingriffs herausgetrennten Knochenstückes am verbliebenen Schädelbein in einem durch das Heraustrennen des Knochenstücks freigelegten Bereich.

Bei Hirnoperationen bedarf es vielfach der Entfernung eines Knochenstücks aus der Schädelkapsel, um dem Operateur den Zugang zum Operationsbereich zu eröffnen. Das vorweg aus der Schädelkapsel herausgesägte Knochenstück muß im Anschluß an die Operation wieder in die Schädelkapsel eingefügt und der Kapsel gegenüber fixiert werden. Die Fixierung des Knochenstücks gegenüber der Schädelkapsel erfolgt bislang durch eine Zusammenfassung des Knochenstücks mit dem verbliebenen Schädelbein durch das Knochenstück und das Schädelbein durchsetzende Stahldrahtschlingen, deren über die Schädelkapsel nach dem Setzen der Drähte vorspringende Enden miteinander verdrillt werden. Hierbei handelt es sich jedoch um eine relativ instabile Zusammenfassung des Knochenstücks mit dem verbliebenen Schädelbein, die das Verwachsen des Knochenstücks mit dem verbliebenen Schädelbein beeinträchtigt und dann auch zu Entzündungen der Kopfhaut führen kann. Darüber hinaus erweist sich diese Art und Weise der Zusammenfassung des Knochenstücks mit dem verbliebenen Schädelbein insofern als nachteilig, als die Stahldrähte beispielsweise bei der postoperativen computertomographischen Kontrolle erhebliche Bildstörungen hervorrufen, die eine sichere Beurteilung der Weichteilstrukturen des Hirns beeinträchtigen. Der Ersatz der Stahldrähte durch nichtresorbierbare körperverträgliche Fäden behebt den zuletztgenannten Nachteil zwar, in Kauf genommen werden muß dabei allerdings eine noch instabilere Fixierung des Knochenstücks gegenüber dem verbliebenen Schädelbein. Die Zusammenfassung des Knochenstücks mit dem verbliebenen Schädelbein mit Hilfe von den Stoß zwischen Knochenstück und Schädelbein überdeckenden, einerseits mit dem Knochenstück und andererseits mit dem verbliebenen Schädelbein verschraubbaren Plättchen aus einem körperverträglichen Metall, wie Titan, die dann auch vor dem Trennschnitt in der Schädelkapsel gesetzte Bohrungen verschließen (EP-A-0 510 390), stellt auch noch keine befriedigende Lösung dar, zumal es sich hierbei um eine umständliche sowie zeitaufwendige und damit kostenintensive Fixierung handelt.

US-A-4002477 offenbart eine Vorrichtung zum Fixieren eines Brustbeins mit einem Stift, einer ersten Scheibe und einer zweiten Scheibe mit einem Durchgangsloch, wobei die zweite Scheibe auf dem Stift aufbringbar und relativ zum Stift verlagerbar und festlegbar ist und wobei die zweite Scheibe mit weiteren Löchern versehen ist.

Ausgehend vom Stand der Technik lag der Erfindung die Aufgabe zugrunde, die Möglichkeit einer einfacheren, mit möglichst geringem Zeitaufwand zu realisierenden Zusammenfassung eines für einen operativen Eingriff aus der Schädelkapsel herausgetrennten Knochenstücks nach dem operativen Eingriff gegenüber dem verbliebenen Schädelbein zu schaffen, die dann auch die gewünschte eindeutige und anhaltende Lagefixierung erbringt.

Die Aufgabe wird mit einer Vorrichtung zum Fixieren eines aus der Schädelkapsel zum Zwecke eines operativen Eingriffs herausgetrennten Knochenstückes in einem am verbliebenen Schädelbein durch das Heraustrennen des Knochenstücks freigelegten Bereich gemäß Anspruch 1 gelöst.

Die im vorausgehenden definierte erfindungsgemäße Fixierungsvorrichtung wird in der Weise angesetzt, daß die Stifte mit ihnen bereits zugeordneter stiftkopfseitiger Scheibe durch eine auf den Durchmesser der Scheibe ausgelegte Aussparung, in der Regel im herausgetrennten Knochenstück, unter die Knochenpartien, der Schaft des Stiftes sich in der Stoßfuge zwischen den Knochenpartien führend, beabstandet über den Umfang des Knochenstücks verteilt werden, auf die aus der Schädelkapsel vorspringenden Stifte sodann die zweite Scheibe aufgesteckt und die zweite Scheibe eine Verkrallung sowohl der ersten als auch der zweiten Scheibe mit den Randbereichen einerseits des verbliebenen Schädelbeins und andererseits des wieder zu integrierenden Knochenstücks angestellt sowie gegenüber dem Stift festgelegt wird.

Ausgestaltungen des erfindungsgemäßen Fixierungsmittels ergeben sich aus den Unteransprüchen 2 bis 21. Als körperverträgliches Metall bietet sich in erster Linie Titan an oder aber eine geeignete Titanverbindung, etwa Ti₆A₆Va. Ein Fixierungsmittel auf Titanbasis erweist sich deshalb als vorteilhaft, weil es die postoperative computertomografische Kontrolle nicht beeinträchtigt. Zur Lagestabilisierung der voreilend auf den Stift aufzusteckenden Scheibe trägt bei, wenn der Übergang vom Kopf des Stiftes in den Schaft konisch mit der Maßgabe ausgebildet ist, das daraus Preßsitz der Scheibe auf dem Stift bei gegen den Kopf des Stiftes anliegender Scheibe resultiert. In Zusammenhang damit ist vorgesehen, daß vom Durchgang der voreilend auf den Stift aufsteckbaren Scheibe radial verlaufende Anschnitte ausgehen, weitergehend kann vorgesehen sein, daß die Scheibe mittig begrenzt eingesenkt ist. Dann auch aus Gründen der Materialersparnis können in den Scheiben zwischen Durchgang und Rand über den Umfang verteilt in regelmäßigen Abständen Aussparungen vorgesehen sein. Die Festlegung des Fixierungsmittels gegenüber den zusammenzufassenden Knochenpartien kann in einer Weise geschehen, die im Prinzip dem Blindnietverfahren entspricht. Hierfür können am Schaft des Stiftes Rasten ausgebildet sein, mit denen die nacheilend auf den Schaft aufsteckbare Scheibe in Richtung auf den Kopf des Stiftes angezogenen Formschluß einzugehen vermag. Erfährt die nacheilend auf den Schaft des Stiftes aufgesteckte Scheibe eine zu ihrer Wölbung gegenläufige Verformung, kommt es allein schon dadurch zu einer Festlegung der Scheibe auf dem Stift, durch Klemmung. Möglich ist auch die Ausführung des Schaftes als Gewindestift, an dem eine Mutter ansetzbar ist, die gegen die nacheilend auf den Stift aufsteckbare Scheibe zur Anlage kommend angezogen wird. In allen Fällen wird die nacheilend auf den Schaft gesteckte Scheibe bis zum Einsenken der an den Scheiben ausgebildeten Zacken in die zusammenzufassenden Knochenpartien verlagert, woraus sich die gewünschte Fixierung des wieder in die Schädelkapsel zu integrierenden Knochenstücks gegenüber den angrenzenden Partien der Schädelkapsel ergibt.

Das neue Fixierungsmittel läßt sich leicht und ohne größeren Zeitaufwand handhaben und erbringt die gewünschte, eindeutige und anhaltende Fixierung des wieder in die Schädelkapsel zu integrierenden, für einen operativen Eingriff aus der Schädelkapsel herausgetrennten Knochenstücks.

In der Zeichnung ist die Erfindung weitergehend erläutert. Es zeigen:
- Figur 1: einen Längsschnitt durch das in Sprengdarstellung dargestellte Fixierungsmittel,
- Figur 2: eine Ausführungsform der voreilend auf den Stift aufzubringenden Scheibe in Richtung des Pfeiles II in Figur 1,
- Figur 3: eine Ausführungsform der nacheilend auf den Stift aufzubringenden Scheibe in Richtung des Pfeiles III in Figur 1,
- Figur 4: die zusammengesetzten Elemente des Fixierungsmittels im Längsschnitt,
- Figur 5: eine den Einsatz des neuen Fixierungsmittels demonstrierende Darstellung,
- Figur 6: einen Schnitt nach Linie VI - VI in Figur 5.

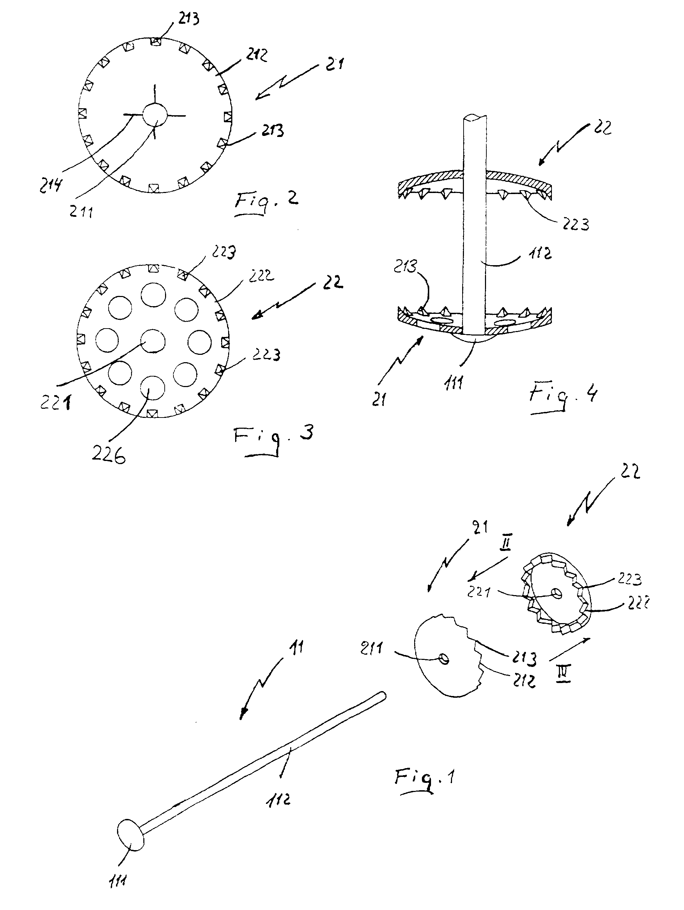

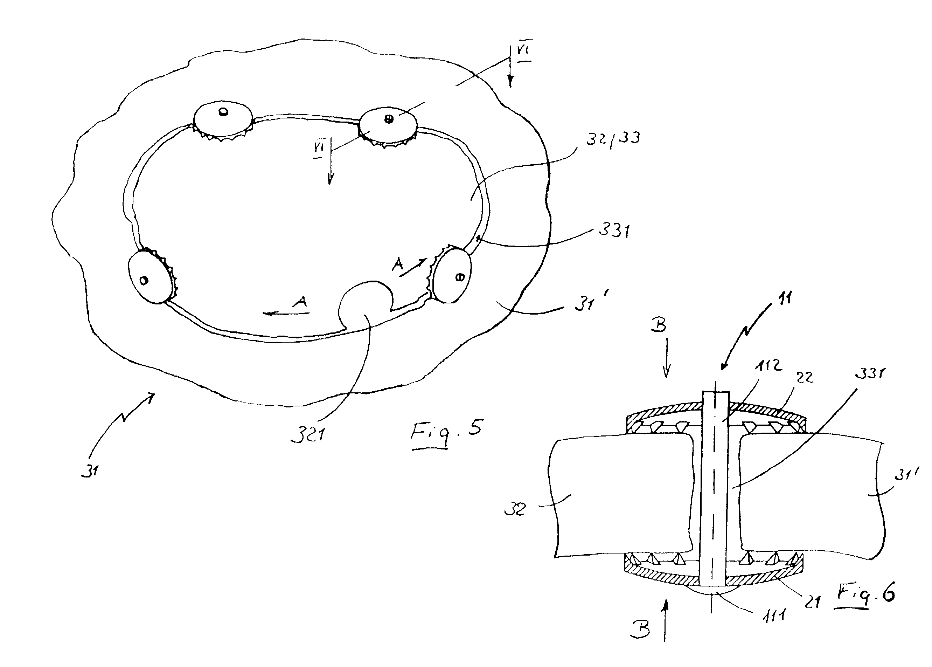

Das Mittel zur Fixierung eines aus der Schädelkapsel herausgetrennten Knochenstücks gegenüber dem verbliebenen Schädelbein nach operativem Eingriff besteht aus dem Stift 11 mit dem Kopf 111 und dem Schaft 112, der vorweg auf den Schaft 112 des Stiftes 11 aufzubringenden, von innen gegen das verbliebene Schädelbein und das damit wieder zusammenzufassende Knochenstück zur Anlage kommenden Scheibe 21 und der dann auch noch auf den Schaft 112 des Stiftes 11 aufzubringenden, von außen gegen das Schädelbein und das damit wieder zusammenzufassende Knochenstück zur Anlage kommenden Scheibe 22. Beide Scheiben sind mittig gelocht, durch die Löcher 221 und 222 erstreckt sich im zusammengesetzten Zustand der Elemente des Fixierungsmittels der Schaft 112 des Stiftes 11. Die Ränder 212, 222 der gewölbten Scheiben 21, 22 sind des weiteren unter Ausbildung von Zacken 213, 223 gezackt. Wie das in Figur 1 angedeutet und aus Figur 4 entnehmbar ist, werden die Scheiben 21 und 22 die gezackten Ränder 212, 222 einander zugewandt auf den Schaft 112 des Stiftes 11 aufgebracht.

Von dem mittigen bezüglich des Querschnitts des Stiftschaftes 112 paßgerechten Durchgangsloch 211, in der Scheibe 21 ausgehende Radialschnitte, angedeutet in Figur 2, dort die Bezugsziffer 214, erbringen bei konischem Übergang vom Kopf 111 des Stiftes 11 in dessen Schaft 112 in wünschenswerter Weise eine eindeutige Zuordnung der Scheibe 21 zum Stift 11, mit der beim Setzen des Fixierungselements das verbliebene Schädelbein und das damit wieder zusammenzufassende Knochenstück unterfahren wird. Die Scheiben 21, 22 können dann auch material- und gewichtssparend im Bereich zwischen Durchgang und Rand über den Umfang verteilt mit Lochungen versehen sein, wie das in Figur 3 angedeutet ist (dort die Bezugsziffer 226). Beide Ausgestaltungen, also die von den Durchgangslöchern ausgehende Anschnitte und die materialsparenden Lochungen, können dann auch gemeinsam vorgesehen sein.

Aus den Figuren 5 und 6 ist die Einsatzweise des neuen Fixierungsmittels zu entnehmen. In Figur 5 ist mit 31 die gewachsene Schädelkapsel bezeichnet, aus der zwecks Ausbildung eines Zugangs zum Gehirn das Knochenstück 32 herausgefräst wurde. Nach Entfernen des Knochenstücks 32 ist das unter der Schädelkapsel liegende Gehirn in diesem Bereich 33 für einen operativen Eingriff zugänglich. Im Anschluß an den vollzogenen Eingriff wird das an seinem Umfang mit einem der Größe der Bestandteil des Fixierungsmittels bildenden, von innen gegen das verbliebene Schädelbein 31' und das damit wieder zusammenzufassende Knochenstück 32 zur Anlage kommenden Scheibe 22 entsprechenden Rücksprung 321 zuzüglich geringfügigen Übermaßes versehene Knochenstück 32 lageorientiert wieder in den freigelegten Bereich 33 der Schädelkapsel eingefügt und aufeinander folgend die Bestandteil des Fixierungsmittels bildenden Stifte 11 mit bereits auf deren Schaft 112 aufgebrachter, von innen gegen das verbliebene Schädelbein 31' und das wieder eingesetzte Knochenstück 32 zur Anlage kommender Scheibe 22 durch den in dem Knochenstück 32 eingearbeiteten Rücksprung 321 in die aus der Fräsung hervorgegangene Stoßfuge 331 überführt und in der Stoßfuge 331 an die Stelle überführt, an der jeweils die Fixierung des Knochenstücks 32 gegenüber dem verbliebenen Schädelbein 31' erfolgen soll (Pfeile A in Fig. 5). An Ort und Stelle wird sodann die zweite, von außen gegen das verbliebene Schädelbein 31' und das damit zusammenzufassende Knochenstück 32 zur Anlage kommende Scheibe 22 auf den Schaft 112 des Stiftes 11 aufgeschoben und zur Anlage gegen das verbliebene Schädelbein 31' und das damit zusammenzufassende Knochenstück 32 gebracht. Am Überstand des Stiftschaftes 112 über die äußere Scheibe 22 wird sodann ein Werkzeug angesetzt, etwa ein Werkzeug nach Art eines Werkzeuges zum Setzen von Blindnieten, mit dem das Fixierungsmittel unter Einsenken der an den Rändern der Scheiben 21, 22 ausgebildeten Zacken 213, 223 in das verbliebene Schädelbein 31' und das Knochenstück 32 gesetzt wird (Pfeile B in Fig. 6). Anschließend wird der danach noch verbleibende Überstand des Stiftschaftes 112 über die äußere Scheibe 22 abgetrennt. Das im vorausgehenden detailliert beschriebene Setzen mit Hilfe eines Werkzeuges nach Art eines Werkzeuges zum Setzen von Blindnieten schließen eine Verklammerung der das verbliebene Schädelbein und das wieder eingesetzte Knochenstück zusammenfassenden Scheiben des Fixierungselementes mit dem Schädelbein und dem Knochenstück über eine Schraubverbindung nicht aus. Dafür ist der Schaft des Bestandteil des Fixierungsmittels bildenden Stiftes dann als Gewindestift ausgebildet, auf dessen Gewinde eine gegen die von außen gegen das verbliebene Schädelbein und das damit wiederum zusammenzufassende Knochenstück zur Anlage kommende, Bestandteil des Fixierungsmittels bildende Scheibe gegen Schädelbein und Knochenstück bis zur Verkrallung beider Scheiben im Schädelbein und am Knochenstück angezogen wird.

Mit solchermaßen über den Umfang des mit dem verbliebenen Schädelbein 31' wieder zusammenzufassenden Knochenstücks 32 verteilt angesetzten erfindungsgemäßen Fixierungsmitteln, wird das Knochenstück 32 gegenüber dem verbliebenen Schädelbein 31' - wie gewünscht - eindeutig fixiert.

## Patentansprüche

1. Vorrichtung zum Fixieren eines aus der Schädelkapsel (31) zum Zwecke eines operativen Eingriffs herausgetrennten Knochenstückes (32) in einem am verbliebenen Schädelbein (31') durch das Heraustrennen des Knochenstücks (32) freigelegten Bereich (33), mit einem einen die Schädelkapsel (31) im Bereich einer Stoßfuge (331) zwischen dem Knochenstück (32) und dem verbliebenen Schädelbein (31') durchsetzenden Schaft (112) aufweisenden Stift (11), einer ersten Scheibe (21) und einer zweiten Scheibe (22), wobei die Vorrichtung aus einem körperverträglichen Material gebildet wird, die erste Scheibe (21) dem Schaft (112) an einem Ende eindeutig zuordenbar ist, die zweite Scheibe (22) in der Mitte mit einem an den Schaft (112) angepaßten Durchgangsloch (221) versehen ist, die zweite Scheibe (22) der ersten Scheibe zugewandt auf den Schaft (112) aufbringbar ist, die zweite Scheibe (22) relativ zum Schaft (112) verlagerbar ist, die zweite Scheibe (22) am Schaft (112) festlegbar ist, die zweite Scheibe (22) in einem Bereich zwischen dem Durchgangsloch (221) und einem Randbereich über den Umfang verteilt in regelmäßiaen Abständen mit weiteren Löchern (226) versehen ist und in der zweiten Scheibe (22) gemeinsam mit den materialsparenden Löchern (226) von dem Durchgangsloch (221) ausgehende Schnitte vorgesehen sind.

2. Vorrichtung nach Anspruch 1, **dadurch gekennzeichnet, daß** die erste Scheibe (21) mittig mit einem Durchgangsloch (211) und in einem Bereich zwischen diesem und einem Randbereich über den Umfang verteilt in regelmäßigen Abständen mit weiteren Löchern (226) versehen ist und daß in der ersten Scheibe (21) gemeinsam mit den materialsparenden Löchern (226) von dem Durchgangsloch (211) ausgehende Schnitte vorgesehen sind.

3. Vorrichtung nach einem der voranstehenden Ansprüche, **dadurch gekennzeichnet, daß** die erste Scheibe (21) gewölbt ist.

4. Vorrichtung nach Anspruch 3, **dadurch gekennzeichnet, daß** die erste Scheibe (21) ein konkave Oberfläche aufweist.

5. Vorrichtung nach einem der voranstehenden Ansprüche, **dadurch gekennzeichnet, daß** die zweite Scheibe (22) gewölbt ist.

6. Vorrichtung nach Anspruch 5, **dadurch gekennzeichnet, daß** die zweite Scheibe (22) ein konkave Oberfläche aufweist.

7. Vorrichtung nach einem der voranstehenden Ansprüche, **dadurch gekennzeichnet, daß** die erste Scheibe (21) in einem Randbereich (212) mit der zweiten Scheibe (22) zugewandten Zacken (213) versehen ist.

8. Vorrichtung nach einem der voranstehenden Ansprüche, **dadurch gekennzeichnet, daß** die zweite Scheibe (22) in einem Randbereich (222) mit der ersten Scheibe (21) zugewandten Zacken (223) versehen ist.

9. Vorrichtung nach einem der voranstehenden Ansprüche, **dadurch gekennzeichnet, daß** das körperverträgliche Material durch ein Metall gebildet wird.

10. Vorrichtung nach Anspruch 9, **dadurch gekennzeichnet, daß** das Metall aus Titan oder einer Titanverbindung gebildet wird.

11. Vorrichtung nach einem der voranstehenden Ansprüche, **dadurch gekennzeichnet, daß** der Schaft (112) an dem einen Ende einen Kopf (111) aufweist.

12. Vorrichtung nach Anspruch 11, **dadurch gekennzeichnet, daß** ein konischer Übergang vom Kopf (111) zum Schaft (112) vorgesehen ist.

13. Vorrichtung nach einem der Ansprüche 11 oder 12, **dadurch gekennzeichnet, daß** der Kopf als Flachkopf (111) ausgebildet ist.

14. Vorrichtung nach einem der Ansprüche 11 bis 13, **dadurch gekennzeichnet, daß** die erste Scheibe (21) an dem Kopf (111) anlegbar ist.

15. Vorrichtung nach einem der voranstehenden Ansprüche, **dadurch gekennzeichnet, daß** eine Formschlußverbindung zwischen der zweiten Scheibe (22) und dem Schaft (112) vorgesehen ist.

16. Vorrichtung nach Anspruch 15, **dadurch gekennzeichnet, daß** am Schaft (112) Rasten ausgebildet sind zum Hinterfangen der auf den Schaft (112) aufgebrachten, in Richtung auf das proximale Ende verlagerten Scheibe (22).

17. Vorrichtung nach einem der voranstehenden Ansprüche, **dadurch gekennzeichnet, daß** von dem Durchgangsloch (211) der ersten Scheibe (21) ausgehende Radialschnitte (214) vorgesehen sind.

18. Vorrichtung nach einem der voranstehenden Ansprüche, **dadurch gekennzeichnet, daß** die erste Scheibe (21) mittig gegenläufig zu ihrer Wölbung eingesenkt ist.

19. Vorrichtung nach einem der voranstehenden Ansprüche, **dadurch gekennzeichnet, daß** der Schaft (112) mit einem Gewinde versehen ist.

20. Vorrichtung nach Anspruch 21, **dadurch gekennzeichnet, daß** die Scheibe (22) auf dem Gewinde gegen die Schädelkapsel (31') und das Knochenstück (32) bis zur Verkrallung der Scheiben (21, 22) in der Schädelkapsel (31') und im Knochenstück (32) anziehbar ist.

21. Vorrichtung nach einem der Ansprüche 21 oder 22, **dadurch gekennzeichnet, daß** eine auf das Gewinde aufschraubbare Mutter vorgesehen ist zum Anziehen der gegen die Mutter zur Anlage kommenden zweiten Scheibe (22) gegen den Schädelknochen (31') und das Knochenstück (32).

## Claims

1. A device for fastening a piece (32) of bone, removed from the cranium (31) for an operation, in a region (33) exposed adjacent the remaining cranial bone (31') through the removal of the bone piece (32), comprising a pin (11) with a shank (112) passing through the cranium (31) in the region of a butt joint (331) between the bone piece (32) and the remaining cranial bone (31'), a first disc (21) and a second disc (22), wherein the device is formed of a body-compatible material, the first disc (21) can be unequivocally associated with one end of the shank (112), the second disc (22) is provided in the middle with a through-hole (221) matched to the shank (112), the second disc (22) can be mounted onto the shank (112) so as to face the first disc, the second disc (22) is displaceable relative to the shank (112), the second disc (22) can be immobilised on the shank (112), the second disc (22) is provided with further holes (226) located in a region between the through-hole (221) and an edge region and distributed at regular intervals over the circumference and incisions issuing from the through-hole (221) are provided in the second disc (22) together with the material-saving holes (226).

2. A device according to claim 1, **characterised in that** the first disc (21) is provided in the middle with a through-hole (211) and in a region between the through-hole (211) and an edge region it is provided with further holes (226) distributed at regular intervals over the circumference and **in that** incisions issuing from the through-hole (211) are provided in the first disc (21) together with the material-saving holes (226).

3. A device according to any one of the preceding claims, **characterised in that** the first disc (21) is convex.

4. A device according to claim 3, **characterised in that** the first disc (21) has a concave surface.

5. A device according to any one of the preceding claims, **characterised in that** the second disc (22) is convex.

6. A device according to claim 5, **characterised in that** the second disc (22) has a concave surface.

7. A device according to any one of the preceding claims, **characterised in that** an edge region (212) of the first disc (22) is provided with teeth (213) facing the second disc (22).

8. A device according to any one of the preceding claims, **characterised in that** an edge region (222) of the second disc (22) is provided with teeth (223) facing the first disc (21).

9. A device according to any one of the preceding claims, **characterised in that** the body-compatible material is formed by a metal.

10. A device according to claim 9, **characterised in that** the metal is formed from titanium or a titanium compound.

11. A device according to any one of the preceding claims, **characterised in that** one end of the shank (112) has a head (111).

12. A device according to claim 11, **characterised in that** a tapering transition from the head (111) to the shank (112) is provided.

13. A device according to one of Claims 11 and 12, **characterised in that** the head is in the form of a flat head (111).

14. A device according to any one of claims 11 to 13, **characterised in that** the first disc (21) can be applied against the head (111).

15. A device according to any one of the preceding claims, **characterised in that** a positive connection between the second disc (22) and the shank (112) is provided.

16. A device according to claim 15, **characterised in that** catches are provided on the shank (112) to catch behind the disc (22) which is mounted onto the shank (112) and which is displaced in the direction of the proximal end.

17. A device according to any one of the preceding claims, **characterised in that** radial incisions (214) issuing from the through-hole (211) of the first disc (21) are provided.

18. A device according to any one of the preceding claims, **characterised in that** the centre of the first disc (21) is depressed in an opposite direction to its convexity.

19. A device according to any one of the preceding claims, **characterised in that** the shank (112) is provided with a thread.

20. A device according to claim 21, **characterised in that** the disc (22) can be screwed down, on the thread, against the cranium (31') and the piece (32) of bone until the discs (21, 22) bite in the cranium (31') and in the piece (32) of bone.

21. A device according to one of claims 21 and 22, **characterised in that** a nut screwable onto the thread is provided to screw down the second disc (22), coming to rest against the nut, against the cranial bone (31') and the piece of bone (32).

## Revendications

1. Dispositif pour fixer un morceau d'os (32), sectionné de la boîte crânienne (31) dans le but d'une intervention chirurgicale, dans une zone (33) dégagée sur l'os crânien (31') restant par le sectionnement du morceau d'os (32), comprenant un goujon (11) présentant une tige (112), qui traverse la boîte crânienne (31) dans la zone d'une jointure (331) entre le morceau d'os (32) et l'os crânien (31') restant, un premier disque (21) et un second disque (22), le dispositif étant fabriqué à partir d'un matériau compatible vis-à-vis du corps ; le premier disque (21) pouvant être associé de manière univoque à la tige (112) à une extrémité ; le second disque (22) étant muni au milieu d'un trou de passage (221) adapté á la tige (112) ; le second disque (22) pouvant être posé sur la tige (112) en étant dirigé vers le premier disque ; le second disque (22) pouvant être déplacé relativement par rapport à la tige (112) ; le second disque (22) pouvant être fixé à la tige (112) ; le second disque (22) étant muni de trous supplémentaires (226) dans une zone entre le trou de passage (221) et une zone de bordure, répartis sur la circonférence à intervalles réguliers ; et des entailles partant du trou de passage (221) étant prévues dans le second disque (22) conjointement avec les trous économisant le matériau (226).

2. Dispositif selon la revendication 1, **caractérisé en ce que** le premier disque (21) est muni au milieu d'un trou de passage (211) et de trous supplémentaires (226) répartis sur la circonférence à intervalles réguliers dans une zone comprise entre ledit trou de passage et une zone de bordure, et des entailles partant du trou de passage (211) sont prévues dans le premier disque (21) conjointement avec les trous économisant le matériau (226).

3. Dispositif selon l'une des revendications précédentes **caractérisé en ce que** le premier disque (21) est bombé.

4. Dispositif selon la revendication 3, **caractérisé en ce que** le premier disque (21) présente une surface concave.

5. Dispositif selon l'une des revendications précédentes **caractérisé en ce que** le second disque (22) est bombé.

6. Dispositif selon la revendication 5, **caractérisé en ce que** le second disque (22) présente une surface concave.

7. Dispositif selon l'une des revendications précédentes **caractérisé en ce que** le premier disque (21) est muni dans une zone de bordure (212) de dentelures (213) dirigées vers le second disque (22).

8. Dispositif selon l'une des revendications précédentes **caractérisé en ce que** le second disque (22) est muni dans une zone de bordure (222) de dentelures (223) dirigées vers le premier disque (21).

9. Dispositif selon l'une des revendications précédentes **caractérisé en ce que** le matériau compatible vis-à-vis du corps est fabriqué à partir de métal.

10. Dispositif selon la revendication 9, **caractérisé en ce que** le matériau est du titane ou un composé à base de titane.

11. Dispositif selon l'une des revendications précédentes **caractérisé en ce que** la tige (112) présente une tête (111) à l'extrémité précitée.

12. Dispositif selon la revendication 11, **caractérisé en ce qu'**une transition conique est prévue de la tête (111) vers la tige (112).

13. Dispositif selon l'une ou l'autre des revendications 11 et 12, **caractérisé en ce que** la tête est réalisée sous forme d'une tête plate (111).

14. Dispositif selon l'une des revendications 11 à 13, **caractérisé en ce que** le premier disque (21) peut être appliqué contre la tête (111).

15. Dispositif selon l'une des revendications précédentes **caractérisé en ce qu'**une liaison par coopération de forme est prévue entre le second disque (22) et la tige (112).

16. Dispositif selon la revendication 15, **caractérisé en ce que** des crans sont ménagés sur la tige (112) pour saisir par l'arrière le disque (22) posé sur la tige (112) et déplacé en direction de l'extrémité proximale.

17. Dispositif selon l'une des revendications précédentes **caractérisé en ce qu'**il est prévu des entailles radiales (214) partant du trou de passage (221) du premier disque (21).

18. Dispositif selon l'une des revendications précédentes **caractérisé en ce que** le premier disque (21) est renfoncé au milieu, en sens inverse à sa courbure.

19. Dispositif selon l'une des revendications précédentes **caractérisé en ce que** la tige (112) est munie d'un filetage.

20. Dispositif selon la revendication 19, **caractérisé en ce que** le disque (22) peut être serré sur le filetage contre l'os crânien (31') et le morceau d'os (32) jusqu'à ce que les disques (21, 22) s'accrochent dans l'os crânien (31') et dans le morceau d'os (32).

21. Dispositif selon l'une ou l'autre des revendications 19 et 20, **caractérisé en ce qu'**il est prévu un écrou pouvant être vissé sur le filetage pour serrer le second disque (22), venant en contact avec l'écrou, contre l'os crânien (31') et le morceau d'os (32).
